(19) Europäisches Patentamt

European Patent Office

Office européen des brevets

(11) **EP 1 129 615 B1**

(12) **EUROPEAN PATENT SPECIFICATION**

(45) Date of publication and mention
of the grant of the patent:
**25.06.2003 Bulletin 2003/26**

(51) Int Cl.7: **A01H 1/04**, A01K 67/02

(21) Application number: **00870030.4**

(22) Date of filing: **01.03.2000**

(54) **Method for combinatorial optimization in plant or animal breeding**

Verfahren zur kombinatorischen Optimierung von Pflanzen- und Tierzucht

Procédé d'optimisation combinatoire pour l'élevage de plantes ou animaux

(84) Designated Contracting States:
**AT BE CH CY DE DK ES FI FR GB GR IE IT LI LU MC NL PT SE**

(43) Date of publication of application:
**05.09.2001 Bulletin 2001/36**

(73) Proprietor: **OM Partners Hortica**
**9810 Eke-Nazareth (BE)**

(72) Inventors:
• **Callewaert, Lieven**
**9810 Eke-Nazareth (BE)**
• **Schepens, Luc**
**8400 Oostende (BE)**

(74) Representative: **Luys, Marie-José A.H. et al**
**Gevers & Vander Haeghen,**
**Intellectual Property House,**
**Brussels Airport Business Park**
**Holidaystraat 5**
**1831 Diegem (BE)**

(56) References cited:
• **DATABASE BIOSIS [Online] BIOSCIENCES INFORMATION SERVICE, PHILADELPHIA, PA, US; AN PREV199900218131, 1999 SCOTT ET AL.: "Relational database system for summarization and interpretation of hard winter wheat regional quality data" XP002146524**
• **DATABASE BIOSIS [Online] BIOSCIENCES INFORMATION SERVICE, PHILADELPHIA, PA, US; AN: PREV200000028385, 1999 REINSCH: "A multiple-species, multiple-project database for genotypes at codominant loci" XP002146525**

**Description**

The present invention relates to a method for breeding plants or animals.

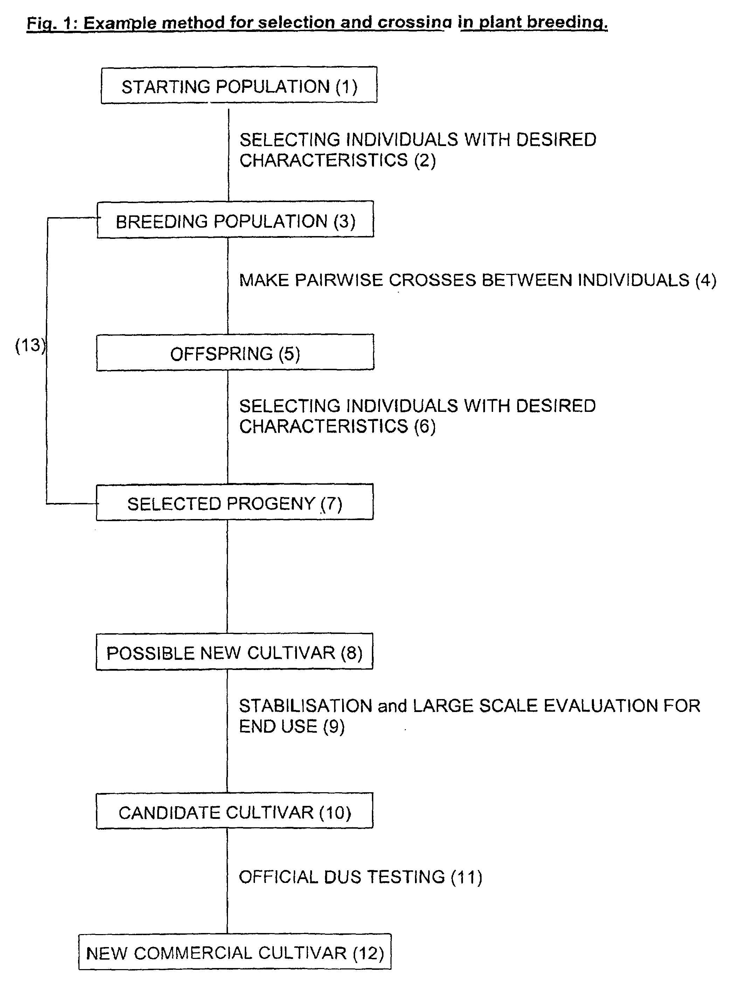

Up to now, only few scientific methods were available to help the breeder in deciding which parents ought to be combined if he wants to develop a new cultivar with altered, often improved characteristics. One of the known methods is based on the study of selection and repetitive crossing of parents from a breeding population, as is shown in figure 1. According to this method, a starting population (1), containing a wide diversity of genotypes of the species concerned, is built up. With this starting population, a large number of experiments is carried out to gather information on the species. Based on the information obtained from those crossing experiments the breeder selects from the starting population, a breeding population (3) of individuals with which the breeding programme is continued. Within the thus obtained breeding population (3), pairs of individuals are crossed (4), and the expression of the characteristic in the progeny (5) obtained from those crossing experiments is evaluated (6). This is the end of the first selection cycle towards a new cultivar. Normally, the variance within the progeny is larger than the variance among the parents. The crossing step is intended to broaden the variance of the population.

After one or more new individuals obtained from the crossing experiments have been selected (7), a new selection cycle can be started as is indicated by the arrow (13). This selection intends to decrease the variance in the breeding population. In case it is possible to reproduce parent individuals (for example through cloning) or with long living individuals (like most animals or perennial plants) parent individuals can be added to the breeding population (3).

A breeding programme mostly comprises a plurality of selection cycles, often 10 to 15 cycles, decreasing and increasing the variance in the breeding population (3), as described above. Thereby, in successive breeding cycles usually those parents will be selected and tested which show the plurality of characteristics aimed at in the new cultivar. A breeding programme usually leads to one or more possible new cultivars (8). After the breeding programme has been completed and before a new cultivar is accepted as a new commercial cultivar, it is investigated whether or not it can be uniformly reproduced in a stable way (9). Official test methods to investigate this are DUS tests (11).

Most often, after one or more new cultivars have been developed, the breeder will continue the breeding programme and perform successive breeding cycles. Thereby additional individuals chosen from the starting population can be added to the breeding population. Most breeders try to maintain their starting population, so as to allow them to return to the beginning of the breeding scheme if necessary, for example if the creation of a new cultivar with completely different characteristics is aimed at.

The method described above gives valuable results. However, because of the large number of crossing experiments needed, the method is rather expensive and time consuming. Also, because of the more subjective approach, the breeder will tend to evaluate individuals on their global assets, rather than on specific characteristics that are possible opportunities or threats to crossings. There is thus a need to find a method for breeding plants or animals with which for a selected species, simultaneously, a plurality of selected characteristics can be optimised, on an individual basis. In other words, a method with which for a selected species, simultaneously a plurality of selected characteristics can be optimised, whereby each characteristic can be handled separately.

This can be achieved with the breeding method of the present invention, which comprises the steps of

a) selecting from a database parents x, y to be evaluated, the database containing data on genotypic and/or phenotypic characteristics $K_k$ for a plurality of characteristics of possible parents $P_z$, whereby z can be varied from 1 to v and v is the maximum number of parents contained in the database;

b) selecting from the database characteristics $K_k$ to be optimised, whereby k = 1 to l, l is the total number of characteristics contained in the database;

c) establishing an objective mathematical function f for calculating from the data on phenotypic and/or genotypic characteristics $K_k$, the over-all combined genetic value Y for each combination of parents x and y and the selected characteristics $K_k$

$$Y = f(x,y) \qquad (100)$$

d) solving a mixed integer linear programming model based upon the objective function f, the solution of which will contain the combined over-all genetic value Y of each possible combination of selected parents x and y.

In step a) either all parents or a limited number of parents contained in the database can be selected to effectuate the breeding method. Step b) allows the breeder to select from a larger number of characteristics $K_k$ contained in the database, either all or only a limited number of characteristics that need to be optimised, and to limit the calculation to the selected characteristics if so desired. In step c) an objective mathematical function is defined. With the thus established mathematical function, the over-all combined genetic value Y can be calculated for every possible combination of two parents (x, y) out of the selected series of parents $P_z$, for the selected characteristics $K_k$, starting from the data on the selected characteristics $K_k$ contained in the database. The over-all combined genetic value is a numerical value obtained from a linear combination of the numerical values of all selected characteristics, phenotypic and genotypic, of the parents considered in the crossing. In step d) the objective mathematical function f defined in step c) is solved, using a mixed integer linear programming model. With this model the combined over-all genetic value Y is calculated for each possible combination of selected parents x and y starting from the data on the selected characteristics $K_k$ contained in the database.

The combination of parents with the highest over-all combined genetic value Y will most often be the combination with which the breeding goal is satisfied best. With the method of the present invention a prediction can be obtained of the extent to which each calculated combination of a pair of individual parents approaches the total image of a presupposed pheno- and/or genotype of progeny. In this way, the method of this invention is situated at position (4) in figure 1, and interferes in the selection of the individuals (2) with which the breeding experiments are carried out.

Thus, the method of this invention assists the breeder in his breeding programme, in that from a large number of possible parents, it is calculated which pairs of individuals ought to be crossed and tested to achieve the new cultivar aimed at. With the method of this invention, information is obtained on those pairs of individuals that ought to be crossed and tested without necessitating a large number of crossing experiments. Surprisingly it has been found that not only those individuals which have the plurality of characteristics in common with the presupposed cultivar turn out to be suitable parents. Also individuals which show only a limited number of the presupposed characteristics, but which in combination show the characteristics aimed at or a plurality thereof, are found to be suitable parents. This means in fact that also those individuals which by themselves possess only a limited number of the characteristics aimed at, and which would therefore be excluded by the breeding method known from the art, may turn out to be suitable parents if they are combined with a suitable partner. As a result, individuals that would otherwise be excluded in the method known from the art will in the method of the present invention be included in the breeding population, so that the number of possible parents can be increased. The inventors are of the opinion that this is due to the fact that the method of this invention takes into account complementarity that may occur between parents.

In T.R. Famula, Theor. Appl. Genet. 1992, 84, pp. 384-389, a theoretical research is described wherein mixed integer linear programming is used for optimising the genetic value of descendants in a crossbreeding programme. A genetic evaluation of one of the parents is made namely the parent capable of generating the largest number of descendants. In T.R. Famula, 1992 an evaluation is made of the sire, because it is capable of producing a large number of descendants. The parent that can only generate the lower number of descendants (mostly the female) is fixed. The average genetic value of the descendants is optimised for one characteristic only, while the change of variation of all further characteristics is constrained to zero. In the method described by Famula, it is nowhere proposed to calculate the combined over-all genetic value of a pair of parents, whereby both individual parents are varied. In other words, in the method described by Famula, for an assumed optimal female, the optimal male is sought. In the method of this invention on the contrary, both the female and male can be varied and are varied until the optimal combination of parents or possible optimal combinations of parents that meet the breeding goal the closest possible, are found. The method of this invention preferably contains the step of maximising the function f. By maximising the function f it becomes immediately clear from the solution of the mathematical function obtained in step d), what combination of individual parents contained in the database gives the best over-all combined genetic value and approaches the presupposed type of descendant as close as possible.

In stead of maximising the function f, the method of this invention may contain the step of minimising the difference between a set ideotype and the calculated combination of individuals.

In the method of this invention, the function f is preferably a linear mathematical function. It has namely been found that the way in which characteristics are inherited by a descendant from its two parents (x, y), can often be described by a linear equation. In practise this could for example mean that descendants from a first parent giving a high crops yield and a second parent giving a low crops yield, will give an intermediate crop yield, like 0.5 (yield 1 + yield 2)*coefficient.

The linear function f preferably is a linear combination of the over-all genetic value of each individual parent:

$$(104) \qquad Y = Y_x + Y_y$$

In the method of this invention, the objective value Y of the combination of two individuals x and y is preferably calculated according to the formula

$$Y = \sum_{k} (g'_k . x_k + h_k . y_k) \qquad (101)$$

wherein k is the index over the selected characteristics, and $g_k$ and $h_k$ are coefficients that may for example depend on the sexe of the parent considered, the type of characteristic considered and the breeding goal.

The parameters $g_k$ and $h_k$ in equation (101) are overall parameters of the characteristics of the individual parents. They can be calculated by combining the value of different parameters including:

- a characteristic dependant parameter describing the goal of the breeder. This means that the breeder can define the relative importance of the selected characteristics with respect to one another
- an inheritance dependant parameter. This parameter defines the relative importance of the genetic determination of the inheritance for a given characteristic
- a sex dependant parameter, which describes the relative inheritance of a characteristic whether inherited either through the male or the female parent
- a crossing scheme dependant parameter. The optimisation method can also be used to simulate different crossing schemes (backcross, three way cross, etc). In those cases an extra parameter may be used to improve the optimisation.
- an individual dependant parameter, with which an individual can be given an extra weight for one or more characteristics.

In formula *(104)*, $Y_x$ and $Y_y$ are preferably represented by

$$Y_x = \sum_{m} \lambda_m \sum_{k} (g_k . x_{km}) \qquad (102)$$

$$Y_y = \sum_{m} \kappa_m \sum_{k} (h_k . y_{km}) \qquad (103)$$

wherein m represents the possible individuals, and $\lambda_m$ and $\kappa_m$ are binary parameters that are either equal to 1 or 0, depending on the fact whether an individual is selected for the calculation or not. Thereby $\lambda_m$, $\kappa_m$ should preferably fulfil the following conditions:

$$\sum_{m} \lambda_m = 1 \qquad (105) \qquad\qquad \sum_{m} \kappa_m = 1 \qquad (106)$$

Equations (105) and (106) are the mathematical translation of the rule that in a pairwise crossing, only one parent x can be combined with one parent y, and constitute the first constraints of the mixed integer linear programming model. A mixed integer-programming algorithm takes care of these extra constraints and of introducing these constraints into the linear programming model.

In the method of this invention preferably

$$\lambda_m < 1 - \kappa_m \qquad \text{for all m} \qquad (107)$$

This makes sure that $x \neq y$, namely that an individual is not crossed with itself. It is namely generally known that crossings between individuals of different origin give better results in a breeding programme than self-pollination can.

In the method of this invention, also either one or both of the following contstraints can be added to the mixed integer linear programming model:

$$C_k \leq C_{kmax} \qquad (108)$$

$$C_k \geq C_{kmin} \qquad (109)$$

wherein $C_k$ is the combined value for a characteristic K, calculated from the data $K_{kx}$ and $K_{ky}$ of that characteristic for each of the parents x and y. The constraints (108) and (109) indicate that, within the function f(x,y), the combined value for a characteristic K should either be equal to or smaller than a maximum value, or equal to or larger than a minimum value. If the calculated $C_k$ does not fulfil this condition, the corresponding combination of parents will be rejected and will not be suggested as a suitable pair of parents for the new cultivar aimed at. The combinations of individuals that fulfil this requirement will be suggested as suitable parents.

Thereby $C_k$ can be defined by the following function:

$$C_k = \sum_m (\lambda_m g_k x_{km}) + \sum_m (\kappa_m h_k y_{km}) \qquad (110)$$

By means of the objective function *(104)* together with the constraints *(105)*, *(106)*, *(107)*, *(108)* and *(109)*, it can be calculated which pairs of crossing partners contained in the database constitute optimal parents to attain a presupposed type of descendant, for a characteristic k.

For some characteristics however, the constraints (108) and (109) may not be the most elegant way to calculate the objective genetic value, for examples in case either the presence or absence of genetic fingerprint data are aimed at, or with qualitative character constraints. In that case the constraints (108) and (109) can be replaced by constraint (111):

$$C_k = C_{kSET} \qquad (111)$$

which indicates that for a characteristic k, the combined genetic value of a pair of possible crossing partners must be equal to a specific value. This can be especially important when qualitative characteristics are considered, which should either be present or not in the combined genetic value of a pair of parents. In that case $C_{kSET}$ should for example be 0 or 1.

By solving a mixed integer linear programming model based on the above identified objective function *(104)* together with the constraints *(105)*, *(106)*, *(107)*, *(108)*, *(109) and (111)* it is possible to calculate and obtain a suggestion on the pairs of individual parents out of a breeding population, that ought to be crossed to approach a presupposed objective in the best way. The result of the method of this invention is that by maximising the function f(x,y), a series of best pairs of parents is suggested which meet the presupposed objective the closest.

However, independent of the model used for calculating the optimal combination of parents, there is always some uncertainty about the result, i.e. the phenotype of the descendants. This is due to the fact that crossings are always carried out with living material. As a consequence, the optimal combination of parents that results from the calculation with the above described method must not necessarily give the best result in a real breeding programme. In practice, the combination that is proposed to be the second best can turn out to be better then the combination proposed as the best one. The method of this invention thus meets the need of providing a method with which the genetic value of a plurality of possible combinations of parents can be calculated, and a plurality of good combinations of possible parents are suggested. This is in accordance with the way breeders set up their crosses. Although a breeder has an idea of two or three excellent crosses within his breeding population, in the state of the art method, he will have to perform a lot of crosses to diminish the risk of not finding the best cultivar. With the method of this invention, the possible best pairs of parents are suggested, so that the risk of not finding the best cultivar can be decreased. In practise, with this invention, the breeder will need less crosses to find the parents allowing to attain his breeding goal.

Also, after an optimal combination of crossing partners $(x_i, y_i)$ has been calculated, it is desirable that this combination is excluded from the next crosses to be calculated, so that following crosses are not identical to former crosses. The individual parents however do not have to be excluded from the next crosses. Namely, it is often possible to cross one individual with a plurality of other individuals (for plants, several flowers can be used; for animals, one paternal parent can produce more offspring with several females). This can be obtained though the equations

$$Z(n+1) = \begin{cases} x_{(n+1)} = x_i \\ \\ y_{(n+1)} = y_i \end{cases} \quad \text{for every } i \ (i: 0 \to n) \qquad (112)$$

$$Z(n+1) \neq \text{TRUE} \qquad \text{for every } i \ (i: 0 \to n) \qquad (113)$$

wherein $Z(n+1)$ is a Boolean, which is TRUE if both equations are fulfilled and FALSE if at least one equation is not fulfilled.

The equations (112) and (113) make the method very flexible to use. Every time an optimal combination of crossing partners has been calculated with the mixed integer linear programming model, that combination is excluded from the next calculations of other possible combinations of parents. This makes it possible to calculate a finite number of combinations of crossing partners, starting from the same breeding population and with the same breeding goals incorporated in the model. Another advantage of this constraint is that the selection of a breeding population out of the starting population can be omitted. The mixed integer linear programming model, through the introducing of the above-described constraints into the model namely makes this selection. In this way it can be avoided that two different populations must be maintained. So, the starting population (1) and the breeding population (3) can be kept as one. An additional advantage is that promising genotypes are not removed accidentally during the selection of the breeding population.

The model described above is the basic model of this invention for calculating the genetic value of a plurality of combinations of possible crossing partners. However, one or more correction factors can be introduced into the above given equations (101), (102), (103), (104), (110), if it is desired to further ameliorate the results of the calculations. Possible examples of correction factors are:

- factors which indicate that the individual considered is either a male or a female,
- a relationship between individuals or characteristics: adding a weight to negatively correlated but desired characteristics
- geographical origin of the individuals: phenotype descriptions may be function of geographic origin, due to environmental conditions Besides , additional information can be incorporated in the model, e.g.
- factors which allow to find an optimal crossing partner for a given phenotype,
- excluding a dominant individual from the calculations
- pre-calculations: in some cases the pre-processing of data may give rise to supplementary data, for example the effect of genetic markers can be calculated via multiple linear regression.

Specific to all these correction factors is that they add additional characteristics to the objective function, change the value of the parameters to be used in the objective function f or add constraints to the characteristics and parameters of the objective function f. This does not affect the generality of the model described above because the invention is not bound to specific characteristics or parameters used, as long as the model remains linear. This is also true for the constraints that are imposed on the characteristics and the parameters.

An analysis of statistical techniques used in breeding programmes has revealed that most often characteristics are inherited by the descendants in a linear way. For this reason a mixed integer linear programming algorithm is used to solve the model.

All mixed integer linear programming models that are used in the method of this invention can be represented in the form of algebraic equations defining objective functions such as equations (102), (103) and (104) and constraint relationships such as the relationships represented by algebraic equations (105), (106), (107), (108), (109), (110), (111), (112) and (113) respectively. A mixed integer linear programming model given by objective function (104) and constraints (105), (106), (107), (108), (109), (110), (111), (112) and (113) may be solved by converting it to a format such as the MPS (Mathematical Programming System) form which has been adopted as a base standard for mathematical programming practitioners. Conversion to the MPS format allows the linear programming model to be read by a variety of commercial mixed integer linear programming systems (e.g. OMP Optimization). This conversion can readily be accomplished by any computer program written for this purpose and does as such not form part of the present invention. The mixed integer linear programming model may also be solved using any of the commercial software systems or combinations thereof, using the simplex or dual simplex method or other suitable algorithms for solution of the linear and mixed integer linear programming models. These methods are for example disclosed in Linear Programming, Vasek Chvatal, W.H. Freeman and Co., Operations Research - Applications and Algorithms, Wayne L. Winston, PWS-Kent Publishing Co.

The solution obtained from the above described mixed integer linear programming model determines the individuals to be crossed in order to obtain a progeny that best fits the idea of the breeder.

In the method of this invention, use can be made of a database which contains breeding characteristics, genotypic and phenoytypic characteristics of possible crossing parents. Thereby available data are collected and stored in a computer database. This can be any commercial or private computer database available to the breeder. The next step consists of data filtering and recalculation, which can be done using a variety of models. In this phase, the data in the database are transformed to render them suitable for use in the optimisation step. Known suitable methods thereto are described in the non-exhaustive list below:

- data encoding: e.g. for genetic data (indicating whether a genetic marker is present or absent) or for qualitative data,
- creating data subsets, if it is desired to limit the starting population. A possible example is the case where it is desired to develop a plant with white flowers, when red is the dominant characteristic. In that case red flowered parents should be excluded from the starting population.
- data standardisation: to fulfil certain computational or formula constraints, for example the scaling of the number, zero average constraint. In that case for example all phenotypical data are averaged around 0 and the standard deviation is set to 1 to simplify the calculation
- combination of data from different origins: creation of common data; e.g. used for parameter-estimation from different sources of data; this may for example take into account the fact that the heritability of a characteristic may vary depending on the environment. The heritability of the characteristic is averaged over all environments, the calculation is carried out based on the average heritability.
- creation of new data through analysis of the basic data: e.g. marker effect estimation through combination of genotypic and phenotypic data
- data estimation: e.g. any form of missing value estimation
- strict data recalculation: e.g. assigning weights to different parts of the data. This can for example be done by attributing a weight to one or more pre-determined markers when calculating the genotypic complementarity.

In the description given above, the invention is mainly oriented to the description of a method for combinatorial optimisation in plant breeding. It should however be clear, and has also been indicated in the description, that the invention is not limited to a plant breeding optimisation method, but may also be used for the combinatorial optimisation in animal breeding. Also, in order to simplify them, the examples given below to illustrate the invention, are related to plant breeding only. They are however also applicable to animal breeding.

Example 1.

As a starting population, use is made of a database containing a population of 20 possible parents $P_z$, plant 1 to plant 20, of maize lines. The breeder has data on 8 phenotypic characteristics K1 to K8 (see table 1), and ten genotypic markers M1 to M10 (see table 2). The whole starting population (plant 1 to plant 20) is used as breeding population. The combined characteristics are subject to the minima as shown in table 6.

In table 3, the data of table 1 are standardised to avoid that one characteristic would determine the result of the calculation and distort the result of the calculation, due to a different valuation of two characteristics.

Genotypic characteristics of the maize lines 1 to 20 are summarised in table 2. Table 2 can be used for calculating genetic distance or genetic similarity. These data can than be used as characteristics in the breeding programme. In this example, the genetic distance is used, i.e. the number of markers that are different for two individual plants. The genetic distances are given in table 4.

It is also possible to combine the data of table 1 and 2. In most cases there are far more genetic markers than there are phenotypic characteristics. This makes it possible to use markers for predicting a value for a characteristic for a certain individual.

The data of table 1 and 2 are inserted in the equations (102) (103) and (104) given above:

$$Y = Y_x + Y_y \qquad (104)$$

$$Y_x = \sum_m \lambda_m \sum_k (g_k . X_{km}) \qquad (102)$$

$$Y_y = \sum_m \kappa_m \sum_k (h_k . y_{km}) \qquad (103)$$

The parameters $g_K$ and $h_k$ in this example consist of two parts. The first part is a weight factor indicating the importance of the characteristic to the breeder and thus the breeding goal. The second part is a factor describing the heritability of the characteristic concerned. This information is summarised in table 5.

The data of table 4 are standardised before they are introduced into the model equation. After standardisation of table 4, the data of table 3, table 4 and table 5 are transformed to an MPS model and the calculation is started. The results are given in table 7. In the first column, the calculated crossings are indicated, together with the over-all combined genetic value for that crossing. Column 2 to 10 indicate the combined genetic value for each individual characteristic. From the results of table 7 it can be seen that plant 18 is a very good crossing partner, although it is not part of the best crossing.

In case the breeder knows in advance that plant 18 is a good crossing partner, he can exclude plant 18 from the calculation, by adding the additional constraints:

$$y_n \neq 18$$

$$x_n \neq 18$$

Table 1:

| Phenotypic characteristics of 20 maize lines. | | | | | | | | |
|---|---|---|---|---|---|---|---|---|
| | K1 | K2 | K3 | K4 | K5 | K6 | K7 | K8 |
| Plant 1 | 18.70 | 2.94 | 36.67 | 554.14 | 71.35 | 194.26 | 1.35 | 25.16 |
| Plant 2 | 6.40 | 2.40 | 36.11 | 445.06 | 60.83 | 157.63 | 1.17 | 21.59 |
| Plant 3 | 3.40 | 2.55 | 30.00 | 422.23 | 49.97 | 174.18 | 1.10 | 22.99 |
| Plant 4 | 14.10 | 2.74 | 40.85 | 478.26 | 78.44 | 191.10 | 1.13 | 26.45 |
| Plant 5 | 19.00 | 3.25 | 45.53 | 509.22 | 85.13 | 208.90 | 1.10 | 26.75 |
| Plant 6 | 18.00 | 1.68 | 58.25 | 503.88 | 62.79 | 171.62 | 1.20 | 23.79 |
| Plant 7 | 18.90 | 2.50 | 16.84 | 613.13 | 73.71 | 200.42 | 1.00 | 31.69 |
| Plant 8 | 18.90 | 3.06 | 42.50 | 537.79 | 69.07 | 208.87 | 1.07 | 29.40 |
| Plant 9 | 3.60 | 2.50 | 38.61 | 514.15 | 57.90 | 158.03 | 1.02 | 19.88 |
| Plant 10 | 4.70 | 2.95 | 22.75 | 717.70 | 71.40 | 198.89 | 1.34 | 27.94 |
| Plant 11 | 18.80 | 2.30 | 38.95 | 534.27 | 68.46 | 200.48 | 1.03 | 21.42 |
| Plant 12 | 16.20 | 2.15 | 34.00 | 532.47 | 64.90 | 199.34 | 1.31 | 26.04 |
| Plant 13 | 18.90 | 2.50 | 36.00 | 484.22 | 60.07 | 184.50 | 1.31 | 16.31 |
| Plant 14 | 6.00 | 2.40 | 24.25 | 574.56 | 65.72 | 179.79 | 1.17 | 36.40 |
| plant 15 | 18.50 | 4.10 | 34.00 | 566.00 | 54.14 | 157.90 | 1.17 | 27.40 |
| plant 16 | 17.70 | 3.10 | 73.25 | 412.75 | 70.72 | 200.80 | 1.27 | 30.51 |
| plant 17 | 16.90 | 2.35 | 35.25 | 549.50 | 68.04 | 172.03 | 0.97 | 21.78 |

Table 1:   (continued)

| Phenotypic characteristics of 20 maize lines. | | | | | | | | |
|---|---|---|---|---|---|---|---|---|
|  | K1 | K2 | K3 | K4 | K5 | K6 | K7 | K8 |
| plant 18 | 18.30 | 2.35 | 36 | 542.11 | 74.07 | 194.13 | 1.47 | 28.13 |
| plant 19 | 6.00 | 2.22 | 34.25 | 541.61 | 68.93 | 176.07 | 1.63 | 21.86 |
| plant 20 | 18.90 | 2.67 | 16.39 | 621.63 | 68.79 | 200.33 | 0.89 | 26.52 |

Table 2:

| Genotypic characteristics of 20 maize lines. | | | | | | | | | | |
|---|---|---|---|---|---|---|---|---|---|---|
|  | M1 | M2 | M3 | M4 | M5 | M6 | M7 | M8 | M9 | M10 |
| plant 1 | 0 | 1 | 0 | 0 | 0 | 0 | 0 | 0 | 1 | 1 |
| plant 2 | 0 | 1 | 1 | 0 | 1 | 0 | 0 | 1 | 1 | 0 |
| plant 3 | 1 | 1 | 0 | 0 | 0 | 0 | 0 | 0 | 1 | 1 |
| plant 4 | 0 | 1 | 1 | 0 | 1 | 1 | 0 | 1 | 1 | 0 |
| plant 5 | 0 | 1 | 0 | 0 | 0 | 1 | 0 | 1 | 1 | 1 |
| plant 6 | 1 | 1 | 0 | 0 | 0 | 1 | 0 | 1 | 1 | 1 |
| plant 7 | 0 | 1 | 0 | 0 | 0 | 0 | 0 | 1 | 0 | 0 |
| plant 8 | 1 | 0 | 0 | 1 | 0 | 0 | 0 | 0 | 1 | 0 |
| plant 9 | 0 | 1 | 1 | 0 | 1 | 0 | 0 | 1 | 1 | 0 |
| plant 10 | 0 | 1 | 0 | 0 | 0 | 1 | 0 | 0 | 0 | 1 |
| plant 11 | 1 | 0 | 1 | 0 | 1 | 1 | 0 | 0 | 0 | 1 |
| plant 12 | 1 | 1 | 0 | 0 | 1 | 0 | 1 | 0 | 1 | 0 |
| plant 13 | 1 | 0 | 0 | 0 | 0 | 0 | 1 | 0 | 1 | 0 |
| plant 14 | 0 | 1 | 0 | 1 | 1 | 0 | 0 | 0 | 1 | 0 |
| plant 15 | 1 | 0 | 0 | 0 | 0 | 0 | 0 | 0 | 1 | 1 |
| plant 16 | 1 | 1 | 0 | 0 | 0 | 1 | 1 | 0 | 1 | 1 |
| plant 17 | 1 | 0 | 1 | 0 | 1 | 0 | 0 | 0 | 1 | 1 |
| plant 18 | 1 | 0 | 0 | 0 | 1 | 0 | 1 | 0 | 0 | 1 |
| plant 19 | 1 | 0 | 1 | 0 | 1 | 0 | 0 | 0 | 1 | 0 |
| plant 20 | 0 | 1 | 0 | 0 | 0 | 1 | 0 | 0 | 0 | 0 |

Table 3:

| Standardised date of 20 maize lines (average per characteristic is 0 and standard deviation is 1). | | | | | | | | |
|---|---|---|---|---|---|---|---|---|
|  | K1 | K2 | K3 | K4 | K5 | K6 | K7 | K8 |
| Plant 1 | 0.73 | 0.60 | 0.01 | 0.30 | 0.51 | 0.46 | 0.88 | -0.10 |
| plant 2 | -1.23 | -0.46 | -0.03 | -1.25 | -0.78 | -1.70 | -0.14 | -0.87 |
| plant 3 | -1.71 | -0.17 | -0.51 | -1.57 | -2.11 | -0.73 | -0.53 | -0.57 |
| plant 4 | 0.00 | 0.21 | 0.34 | -0.78 | 1.37 | 0.27 | -0.36 | 0.18 |
| plant 5 | 0.78 | 1.21 | 0.70 | -0.33 | 2.19 | 1.33 | -0.53 | 0.24 |
| plant 6 | 0.63 | -1.88 | 1.68 | -0.41 | -0.54 | -0.88 | 0.03 | -0.39 |

Table 3:   (continued)

| **Standardised date of 20 maize lines** (average per characteristic is 0 and standard deviation is 1). | | | | | | | | |
|---|---|---|---|---|---|---|---|---|
|  | K1 | K2 | K3 | K4 | K5 | K6 | K7 | K8 |
| plant 7 | 0.77 | -0.27 | -1.53 | 1.14 | 0.79 | 0.82 | -1.10 | 1.30 |
| plant 8 | 0.77 | 0.84 | 0.46 | 0.07 | 0.23 | 1.32 | -0.70 | 0.81 |
| plant 9 | -1.68 | -0.27 | 0.16 | -0.26 | -1.14 | -1.68 | 0.03 | -1.23 |
| plant 10 | -1.51 | 0.62 | -1.07 | 2.63 | 0.51 | 0.73 | 0.83 | 0.50 |
| plant 11 | 0.75 | -0.66 | 0.19 | 0.02 | 0.15 | 0.83 | -0.93 | -0.90 |
| plant 12 | 0.33 | -0.96 | -0.20 | 0.00 | -0.28 | 0.76 | 0.66 | 0.09 |
| plant 13 | 0.77 | 0.27 | -0.04 | -0.69 | -0.88 | -0.12 | 0.66 | -2.00 |
| plant 14 | -1.30 | -0.46 | 0.95 | 0.60 | -0.18 | -0.39 | -0.14 | 2.14 |
| plant 15 | 0.71 | 2.89 | -0.20 | 0.47 | -1,60 | -1.69 | -0.14 | 0.38 |
| plant 16 | 0.58 | 0.92 | 2.85 | -1.71 | 0.43 | 0.85 | 0.43 | 1.05 |
| plant 17 | 0.44 | -0.56 | -0.10 | 0.24 | 0.10 | -0.85 | -1.27 | -.083 |
| plant 18 | 0.68 | -0.56 | -0.04 | 0.13 | 0.84 | 0.45 | 1.57 | 0.54 |
| plant 19 | -1.30 | -0.82 | -0.18 | 0.13 | 0.21 | -0.61 | 2.47 | -0.81 |
| plant 20 | 0.77 | 0.07 | -1.56 | 1.27 | 0.19 | 0.82 | -1.72 | 0.19 |

Table 4: Genetic Distance

| | P1 | P2 | P3 | P4 | P5 | P6 | P7 | P8 | P9 | P10 | P11 | P12 | P13 | P14 | P15 | P16 | P17 | P18 | P19 | P20 |
|---|---|---|---|---|---|---|---|---|---|---|---|---|---|---|---|---|---|---|---|---|
| Plant 1 | 0 | 4 | 1 | 5 | 2 | 3 | 3 | 4 | 4 | 2 | 6 | 4 | 4 | 3 | 2 | 3 | 4 | 5 | 5 | 3 |
| Plant 2 | 4 | 0 | 5 | 1 | 6 | 5 | 3 | 6 | 0 | 6 | 6 | 4 | 6 | 3 | 6 | 7 | 4 | 7 | 3 | 5 |
| Plant 3 | 1 | 5 | 0 | 6 | 3 | 2 | 4 | 3 | 5 | 3 | 5 | 3 | 3 | 4 | 1 | 2 | 3 | 4 | 4 | 4 |
| Plant 4 | 5 | 1 | 6 | 0 | 5 | 4 | 4 | 7 | 1 | 5 | 5 | 5 | 7 | 4 | 7 | 6 | 5 | 8 | 4 | 4 |
| Plant 5 | 2 | 6 | 3 | 5 | 0 | 3 | 3 | 6 | 6 | 0 | 4 | 6 | 6 | 5 | 4 | 3 | 6 | 5 | 7 | 1 |
| Plant 6 | 3 | 5 | 2 | 4 | 3 | 0 | 4 | 5 | 5 | 3 | 5 | 5 | 5 | 6 | 3 | 2 | 5 | 6 | 6 | 4 |
| Plant 7 | 3 | 3 | 4 | 4 | 3 | 4 | 0 | 5 | 3 | 3 | 7 | 5 | 5 | 4 | 5 | 6 | 7 | 6 | 6 | 2 |
| Plant 8 | 4 | 6 | 3 | 7 | 6 | 5 | 5 | 0 | 6 | 6 | 6 | 4 | 2 | 3 | 2 | 5 | 4 | 5 | 3 | 5 |
| Plant 9 | 4 | 0 | 5 | 1 | 6 | 5 | 3 | 6 | 0 | 6 | 6 | 4 | 6 | 3 | 6 | 7 | 4 | 7 | 3 | 5 |
| Plant 10 | 2 | 6 | 3 | 5 | 0 | 3 | 3 | 6 | 6 | 0 | 4 | 6 | 6 | 5 | 4 | 3 | 6 | 5 | 7 | 1 |
| Plant 11 | 6 | 6 | 5 | 5 | 4 | 5 | 7 | 6 | 6 | 4 | 0 | 6 | 6 | 7 | 4 | 5 | 2 | 3 | 3 | 5 |
| Plant 12 | 4 | 4 | 3 | 5 | 6 | 5 | 5 | 4 | 4 | 6 | 6 | 0 | 2 | 3 | 4 | 3 | 4 | 3 | 3 | 5 |
| Plant 13 | 4 | 6 | 3 | 7 | 6 | 5 | 5 | 2 | 6 | 6 | 6 | 2 | 0 | 5 | 2 | 3 | 4 | 3 | 5 | 5 |
| Plant 14 | 3 | 3 | 4 | 4 | 5 | 6 | 4 | 3 | 3 | 5 | 7 | 3 | 5 | 0 | 5 | 6 | 5 | 6 | 4 | 4 |
| Plant 15 | 2 | 6 | 1 | 7 | 4 | 3 | 5 | 2 | 6 | 4 | 4 | 4 | 2 | 5 | 0 | 3 | 2 | 3 | 3 | 5 |
| Plant 16 | 3 | 7 | 2 | 6 | 3 | 2 | 6 | 5 | 7 | 3 | 5 | 3 | 3 | 6 | 3 | 0 | 5 | 4 | 6 | 4 |
| Plant 17 | 4 | 4 | 3 | 5 | 6 | 5 | 7 | 4 | 4 | 6 | 2 | 4 | 4 | 5 | 2 | 5 | 0 | 3 | 1 | 7 |
| Plant 18 | 5 | 7 | 4 | 8 | 5 | 6 | 6 | 5 | 7 | 5 | 3 | 3 | 3 | 6 | 3 | 4 | 3 | 0 | 4 | 6 |
| Plant 19 | 5 | 3 | 4 | 4 | 7 | 6 | 6 | 3 | 3 | 7 | 3 | 3 | 5 | 4 | 3 | 6 | 1 | 4 | 0 | 6 |
| Plant 20 | 3 | 5 | 4 | 4 | 1 | 4 | 2 | 5 | 5 | 1 | 5 | 5 | 5 | 4 | 5 | 4 | 7 | 6 | 6 | 0 |

## Table 5: Importance and heritability per character are forming the objective parameter.

|  | K1 | K2 | K3 | K4 | K5 | K6 | K7 | K8 | G.D.* |
|---|---|---|---|---|---|---|---|---|---|
| Weight | 1.5 | 0.5 | 0.6 | 1.0 | 1.5 | 1.2 | 2.0 | 2.5 | 2.0 |
| Heritability | 0.4 | 0.6 | 0.6 | 0.7 | 0.6 | 0.8 | 0.4 | 0.8 | 0.5 |
| Parameter | 0.6 | 0.3 | 0.36 | 0.7 | 0.9 | 0.96 | 0.8 | 2.0 | 1.0 |

*GD = the genetic distance

## Table 6: Minimum values imposed to the combined value for each individual characteristic.

|  | C1 | C2 | C3 | C4 | C5 | C6 | C7 | C8 | G.D.* |
|---|---|---|---|---|---|---|---|---|---|
| minimum | -0.50 | -0.55 | -0.80 | -0.85 | 0 | 0 | -0.65 | 0 | -0.60 |

EP 1 129 615 B1

## Table 7: The 15 best combinations of parents (female – male)

| Crossing (value) | C1 | C2 | C3 | C4 | C5 | C6 | C7 | C8 | Gen.Dist. |
|---|---|---|---|---|---|---|---|---|---|
| Plant 14 - Plant 16 (4.74193402) | -0.36 | 0.23 | 0.95 | -0.56 | 0.12 | 0.23 | 0.15 | 1.73 | 1.03 |
| Plant 18 - Plant 4 (4.64735301) | 0.34 | -0.18 | 0.15 | -0.32 | 1.11 | 0.36 | 0.60 | 0.36 | 2.13 |
| Plant 14 - Plant 18 (4.61329059) | -0.31 | -0.51 | -0.50 | 0.36 | 0.33 | 0.03 | 0.71 | 1.47 | 1.03 |
| Plant 8 - Plant 10 (4.54791015) | -0.37 | 0.73 | -0.30 | 1.35 | 0.37 | 1.03 | 0.06 | 0.65 | 1.03 |
| Plant 18 - Plant 5 (4.47862576) | 0.73 | 0.32 | 0.33 | -0.10 | 1.52 | 0.89 | 0.52 | 0.39 | 0.48 |
| Plant 8 - Plant 16 (4.26515742) | 0.68 | 0.88 | 1.66 | -0.82 | 0.33 | 1.08 | -0.14 | 0.93 | 0.48 |
| Plant 14 - Plant 5 (4.2231424) | -0.26 | 0.37 | -0.13 | 0.13 | 1.00 | 0.47 | -0.33 | 1.33 | 0.48 |
| Plant 18 - Plant 10 (4.184193332) | -0.42 | 0.03 | -0.55 | 1.38 | 0.67 | 0.59 | 1.20 | 0.52 | 0.48 |
| Plant 8 - Plant 18 (4.13651400) | 0.73 | 0.14 | 0.21 | 0.10 | 0.53 | 0.89 | 0.43 | 0.68 | 0.48 |
| Plant 8 - Plant 4 (3.915093308) | 0.39 | 0.52 | 0.40 | -0.35 | 0.80 | 0.80 | -0.53 | 0.49 | 1.58 |
| Plant 18 - Plant 16 (3.90144060) | 0.63 | 0.18 | 1.40 | -0.79 | 0.63 | 0.65 | 1.00 | 0.79 | -0.06 |
| Plant 12 - Plant 5 (3.61836517) | 0.56 | 0.13 | 0.25 | -0.17 | 0.95 | 1.04 | 0.06 | 0.17 | 1.03 |
| Plant 18 - Plant 1 (3.5248346) | 0.71 | 0.02 | -0.01 | 0.22 | 0.67 | 0.46 | 1.23 | 0.22 | 0.48 |
| Plant 18 - Plant 20 (3.33764709) | 0.73 | -0.25 | -0.80 | 0.70 | 0.52 | 0.64 | -0.08 | 0.37 | 1.03 |
| Plant 4 - Plant 5 (3.16122809) | 0.39 | 0.71 | 0.52 | -0.56 | 1.78 | 0.80 | -0.45 | 0.21 | 0.48 |

**Claims**

1. A method for breeding animals or plants comprising the steps of

a) selecting from a database parents x, y to be evaluated, the database containing data on genotypic and/or phenotypic characteristics $K_k$ for a plurality of characteristics of possible parents $P_z$, whereby z can be varied from 1 to v and v is the maximum number of parents contained in the database;

b) selecting from the database characteristics $K_k$ to be optimised, whereby k = 1 to l, l is the total number of characteristics contained in the database;

c) establishing an objective mathematical function f for calculating from the data on phenotypic and/or genotypic characteristics $K_k$, an over-all combined genetic value Y for each combination of parents x and y and the selected characteristics $K_k$, wherein

$$Y = f(x, y) \qquad (100)$$

d) solving a mixed integer linear programming model based on the objective function f, and calculating the combined over-all genetic value Y of each possible combination of selected parents x and y.

2. A method as claimed in claim 1, **characterised in that** the method comprises the step of maximising the function f.

3. A method as claimed in claim 1 or 2, **characterised in that** the function f is preferably a linear mathematical function.

4. A method as claimed in any one of claims 1 to 3, **characterised in that**

$$Y = Y_x + Y_y \qquad (104)$$

wherein $Y_x$ is the over-all genetic value of parent x and Yy is the over-all genetic value of parent y.

5. A method as claimed in any one of claims 1 to 4, **characterised in that**

$$Y = \sum_k \left( g_k.x_k + h_k.y_k \right) \qquad (101)$$

wherein $g_k$ and $h_k$ are coefficients which may depend on the sexe of the parent considered, the type of characteristic taken into consideration, and the breeding goal, and k is the index over the selected characteristics.

6. A method as claimed in claim 4 or 5, **characterised in that**

$$Y_x = \sum_m \lambda_m \sum_k \left( g_k.x_{km} \right) \qquad (102)$$

$$Y_y = \sum_m \kappa_m \sum_k \left( h_k.y_{km} \right) \qquad (103)$$

wherein $\lambda_m$ and $\kappa_m$ are binary parameters that are either equal to 1 or 0, depending on the fact whether an individual is selected for the calculation or not.

7. A method as claimed in claim 6, **characterised in that**

$$\sum \lambda_m = 1 \quad (105) \qquad \sum_m \kappa_m = 1 \qquad (106)$$

**8.** A method as claimed in claim 6 or 7,
**characterised in that**

$$\lambda_m < 1 - \kappa_m, \text{ for all m.} \quad (107)$$

**9.** A method as claimed in any one of claims 1 to 8, **characterised in that**

$$C_k \leq C_{kmax} \quad (108)$$

$$Ck \geq C_{kmin} \quad (109)$$

wherein $C_k$ is the combined value for a characteristic K, calculated from the data $K_{kx}$, $K_{ky}$ of characteristic K for each of the individuals of a pair of parents x, y.

**10.** A method as claimed in any one of claims 1-9, **characterised in that**

$$(112) \quad Z(n+1) = \begin{cases} X_{(n+1)} = X_i \\ \\ y_{(n+1)} = y_i \end{cases} \text{for every i (i: 0 -> n)}$$

$$Z(n+1) \neq TRUE \quad \text{for every i (i: 0 -> n)} \quad (113)$$

**Patentansprüche**

**1.** Verfahren zum Züchten von Tieren oder Pflanzen, umfassend folgende Schritte:

a) Auswählen aus einer Datenbank von Eltern x, y, die ausgewertet werden sollen, wobei die Datenbank Daten über genotypische und/oder phänotypische Merkmale $K_k$ für mehrere Merkmale möglicher Eltern $P_z$ enthält, wobei z von 1 bis v variieren kann und v die maximale Anzahl von Eltern ist, die in der Datenbank enthalten sind;

b) Auswählen aus der Datenbank von Merkmalen $K_k$, die optimiert werden sollen, wobei k = 1 bis l, wobei l die Gesamtanzahl von Merkmalen ist, die in der Datenbank enthalten sind;

c) Aufstellen einer mathematischen Zielfunktion f zum Errechnen aus den Daten über phänotypische und/oder genotypische Merkmale $K_k$ eines kombinierten genetischen Gesamtwerts Y für jede Kombination von Elternteilen x und y und die ausgewählten Merkmale $K_k$, wobei

$$Y = f(x,y) \quad (100)$$

d) Lösen eines gemischten ganzzahligen linearen Programmiermodells auf Grundlage der Zielfunktion f und Errechnen des kombinierten genetischen Gesamtwerts Y jeder möglichen Kombination von ausgewählten Elternteilen x und y.

2. Verfahren nach Anspruch 1, **dadurch gekennzeichnet, daß** das Verfahren den Schritt des Maximierens der Funktion f umfaßt.

3. Verfahren nach einem der Ansprüche 1 oder 2, **dadurch gekennzeichnet, daß** die Funktion f vorzugsweise eine lineare mathematische Funktion ist.

4. Verfahren nach einem der Ansprüche 1 bis 3, **dadurch gekennzeichnet, daß**

$$Y = Yx + Yy \qquad (104)$$

wobei $Y_x$ der genetische Gesamtwert von Elternteil x und $Y_y$ der genetische Gesamtwert von Elternteil y ist.

5. Verfahren nach einem der Ansprüche 1 bis 4, **dadurch gekennzeichnet, daß**

$$Y = \sum_k (g_k . x_k + h_k . y_k) \qquad (101)$$

wobei $g_k$ und $h_k$ Koeffizienten sind, die vom Geschlecht des in Betracht kommenden Elternteils, von der Merkmalart, die in Betracht gezogen wird, und vom Zuchtziel abhängig sein können, und k der Index über die ausgewählten Merkmale ist.

6. Verfahren nach einem der Ansprüche 4 oder 5, **dadurch gekennzeichnet, daß**

$$Yx = \sum_m \lambda_m \sum_k (g_k . x_{km}) \qquad (102)$$

$$Yy = \sum_m k_m \sum_k (h_k . y_{km}) \qquad (103)$$

wobei $\lambda_m$ und $k_m$ binäre Parameter sind, die entweder gleich 1 oder gleich 0 sind, abhängig von der Tatsache, ob ein Individuum für die Berechnung ausgewählt ist oder nicht.

7. Verfahren nach Anspruch 6, **dadurch gekennzeichnet, daß**

$$\sum_m \lambda_m = 1 \quad (105) \qquad \sum_m \kappa_m = 1 \qquad (106)$$

8. Verfahren nach einem der Ansprüche 6 oder 7, **dadurch gekennzeichnet, daß**

$$\lambda_m < 1 - K_m, \text{ für alle m.} \qquad (107)$$

9. Verfahren nach einem der Ansprüche 1 bis 8, **dadurch gekennzeichnet, daß**

$$C_k \leq C_{kmax} \qquad (108)$$

$$C_k \geq C_{kmin} \qquad (109)$$

wobei $C_k$ der kombinierte Wert für ein Merkmal K ist, der aus den Daten $K_{kx}$, $K_{ky}$ von Merkmal K für jedes der Individuen eines Paars von Eltern x,y errechnet wird.

10. Verfahren nach einem der Ansprüche 1 bis 9, **dadurch gekennzeichnet, daß**

$$Z(n+1) = X_{(n+1)} = X_i$$

für alle i(i:0->n) $\qquad (112)$

$$Z(n+1) = Y_{(n+1)} = Y_i$$

$Z(n+1) \neq$ WAHR $\qquad$ für alle i(i:0->n) $\qquad (113)$

**Revendications**

1. Procédé d'élevage d'animaux ou de culture de plantes comprenant les étapes de

a) sélectionner dans une base de données des parents x,y à évaluer, la base de données contenant des données relatives aux caractéristiques $K_k$ de génotype et/ou de phénotype d'une pluralité de parents $P_z$ possibles, où z varie de 1 à v, v étant le nombre maximum de parents contenus dans la base de données ;
b) sélectionner dans la base de données des caractéristiques $K_k$ à optimiser, où k = 1 à I, I étant le nombre total de caractéristiques contenues dans la base de données ;
c) établir une fonction mathématique objective f pour calculer à partir des données relatives aux caractéristiques $K_k$ de phénotypes et/ou génotypes, une valeur génétique combinée globale Y pour chaque combinaison de parents x et y et la caractéristique $K_k$ sélectionnée, où

$$Y=f(x,y) \qquad (100)$$

d) résoudre un modèle de programmation linéaire intégré mixte basé sur la fonction objective f et calculer la valeur génétique combinée globale Y de chaque combinaison possible de parents sélectionnés x et y.

2. Procédé selon la revendication 1, **caractérisé en ce qu'**il comprend une étape de maximisation de la fonction f.

3. Procédé selon l'une des revendications 1 ou 2, **caractérisé en ce que** la fonction f est de préférence une fonction mathématique linéaire.

4. Procédé selon l'une quelconque des revendications 1 à 3, **caractérisé en ce que**

$$Y = Y_x + Y_y \qquad (104)$$

où $Y_x$ est la valeur génétique globale du parent x et $Y_y$ la valeur génétique globale du parent y.

5. Procédé selon l'une quelconque des revendications 1 à 4, **caractérisé en ce que**

$$Y = \sum_k \left( g_k . x_k + h_k . y_k \right) \qquad (101)$$

où $g_k$ et $h_k$ sont des coefficients qui peuvent dépendre du sexe du parent considéré, du type de caractéristique prise en considération et du but de l'élevage et k est l'index au travers des caractéristiques sélectionnées.

6. Procédé selon l'une des revendications 4 ou 5, **caractérisé en ce que**

$$Y_x = \sum_m \lambda_m \sum_k \left( g_k . x_{km} \right) \qquad (102)$$

$$Y_y = \sum_m \kappa_m \sum_k \left( h_k . y_{km} \right) \qquad (103)$$

où $\lambda_m$ et $\kappa_m$ sont des paramètres binaires qui sont égaux à 0 ou 1, selon le fait qu'un individu est sélectionné pour le calcul ou non.

7. Procédé selon la revendication 6, **caractérisé en ce que**

$$\sum_m \lambda_m = 1 \quad (105) \qquad\qquad \sum_m \kappa_m = 1 \quad (106)$$

8. Procédé selon la revendication 6 ou 7, **caractérisé en ce que**

$$\lambda_m < 1 - \kappa_m, \text{ pour tout m.} \qquad (107)$$

9. Procédé selon l'une quelconque des revendications 1 à 8, **caractérisé en ce que**

$$C_k \leq C_{kmax} \qquad (108)$$

$$C_k \geq C_{kmin} \qquad (109)$$

où $C_k$ est la valeur combinée pour une caractéristique K, calculée à partir des données $K_{kx}$, $K_{ky}$ de la caractéristique K pour chaque individu d'une paire de parents x,y.

10. Procédé selon l'une quelconque des revendications 1-9, **caractérisé en ce que**

$$Z_{(n+1)} = X_{(n+1)} = X_i$$

$$\text{pour tout i } (i = 0 \rightarrow n) \qquad (112)$$

$$Z_{(n+1)} = y_{(n+1)} = y_i$$

$$Z_{(n+1)} \neq \text{VRAI pour tout i } (i = 0 \rightarrow n) \qquad (113)$$

**Fig. 1: Example method for selection and crossing in plant breeding.**

```
              ┌──────────────────────────────┐
              │   STARTING POPULATION (1)     │
              └──────────────────────────────┘
                           │
                           │   SELECTING INDIVIDUALS WITH DESIRED
                           │   CHARACTERISTICS (2)
                           │
              ┌──────────────────────────────┐
      ┌───────│   BREEDING POPULATION (3)     │
      │       └──────────────────────────────┘
      │                    │
      │                    │   MAKE PAIRWISE CROSSES BETWEEN INDIVIDUALS (4)
      │                    │
(13)  │       ┌──────────────────────────────┐
      │       │       OFFSPRING (5)           │
      │       └──────────────────────────────┘
      │                    │
      │                    │   SELECTING INDIVIDUALS WITH DESIRED
      │                    │   CHARACTERISTICS (6)
      │                    │
      │       ┌──────────────────────────────┐
      └───────│    SELECTED PROGENY (7)       │
              └──────────────────────────────┘
                           │
                           │
                           │
              ┌──────────────────────────────┐
              │  POSSIBLE NEW CULTIVAR (8)    │
              └──────────────────────────────┘
                           │
                           │   STABILISATION and LARGE SCALE EVALUATION FOR
                           │   END USE (9)
                           │
              ┌──────────────────────────────┐
              │   CANDIDATE CULTIVAR (10)     │
              └──────────────────────────────┘
                           │
                           │   OFFICIAL DUS TESTING (11)
                           │
              ┌──────────────────────────────┐
              │ NEW COMMERCIAL CULTIVAR (12)  │
              └──────────────────────────────┘
```